# EUROPEAN PATENT APPLICATION

(11) **EP 3 114 962 A1**
(43) Date of publication of application: **11.01.2017**
(21) Application number: 15196410.3
(22) Date of filing: 26.11.2015
(51) Int. Cl.: A45D 33/00, A45D 34/00

(54) **CONTAINER OF FLUID COSMETIC PRODUCT WITH SUPERABSORBENT POLYMER**

(30) Priority: 06.07.2015 IT UB20151929
(71) Applicant: Intercos S.p.A., 20122 Milano (IT)
(72) Inventor: IBRAHIM, Hanno, 20064 Gorgonzola MI (IT); LATTANZI, Giuseppe, 20020 Misinto MB (IT); CARBONARA, Lucia, 20139 Milano (IT); VALSESIA, Patrizia, 23885 Calco LC (IT)
(74) Representative: Mittler, Enrico

(57) **Abstract**

A container (1) of cosmetic products is described, comprising a support (2), a tank (3), an insert (4) and an application sponge (5). Said insert (4) is provided with a canvas (42) put in contact with a cosmetic product inserted in the tank (3). The cosmetic product comprises a cosmetic formulation combined with superabsorbent polymers (Figure 1).

## Description

The present invention relates to a container of fluid cosmetic product with superabsorbent polymer.

Modern life leads people to spend many hours of the day away from home. Work, educational or leisure commitments force increasingly more people to return home only after many hours of absence. The need to bring everything along in order to spend a serene day away from home has stimulated the imagination of many companies that have begun to produce more and more "life-saver" kits to bring along.

The world of cosmetics is certainly not exempt from these needs. There have been innumerable preparations and pocket compact containers marketed in recent decades. In the past, compact containers of cosmetic products were almost exclusively dedicated to powder products such as blush, powder and the like which allowed the touching up even outside home. For more delicate products, generally fluid preparations, it became very difficult to put them in containers for use outside home.

Several attempts have been made to use liquid cosmetic products in portable containers but to date, this remains an unresolved issue, especially due to the difficulty of managing a liquid product inside a portable container.

EP0528705 describes a spongy element placed inside a container with lid and preferably with flexible synthetic open-cell foam, such as polyurethane. The spongy element is impregnated with a fluid cosmetic product and is topped by a grid consisting of a woven fabric or other porous flexible element held in compressed position on top of the spongy element.

Disadvantageously, using a spongy tissue such as polyurethane, impregnated with a fluid cosmetic composition does not allow an optimal dosage of the cosmetic product to be applied. This may lead to a too abundant application of the product following a non-optimal pressure on the sponge impregnated with the cosmetic fluid.

WO2012/128589 describes a cosmetic product including a polyether-based urethane foam impregnated with a cosmetic composition, in particular in the form of an emulsion.

WO2009/116817 describes a cosmetic product with a composition adapted to block ultraviolet rays which is impregnated in an expanded polyurethane foam placed inside the container. The polyurethane composition has an expanded cell structure.

Disadvantageously, the cosmetic products described are generally prepared as emulsions and then placed into containers with urethane foams. Emulsions are not stable preparations over time and tend to deteriorate, losing or significantly changing the features of the product initially inserted into the packaging. Moreover, urethane foams do not allow an optimal distribution and dispensing of the cosmetic product.

The object of the present invention is to have a container able to deliver a balanced portion of cosmetic product.

Another object is to have a container for cosmetic products able to maintain the features of the product contained stable over time.

A further object is to have a container for cosmetic products able to allow the use of cosmetic products also outside home and in places with no water.

An additional object is to have a packaging for cosmetic products that allows carrying the cosmetic product without risk of spillage.

Yet another object is to have a container for cosmetic products that allows applying the cosmetic product evenly, without dirtying one's hands.

According to the invention, such objects are achieved by the container described in claim 1.

These and other features of the present invention will appear more clearly from the following detailed description of the practical embodiments thereof, made by way of a non-limiting example with reference to the accompanying drawings, in which:

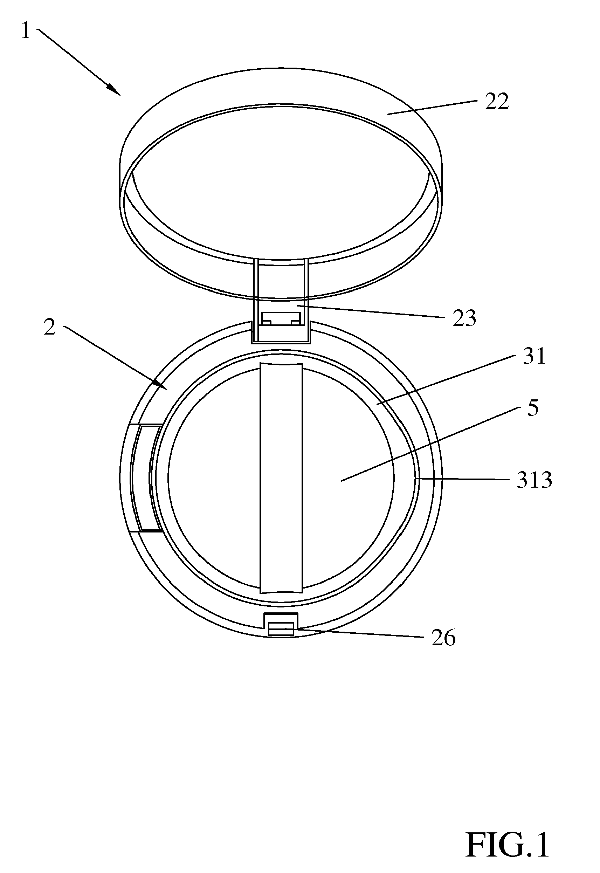

Figure 1 shows a perspective view of a container for cosmetic products;

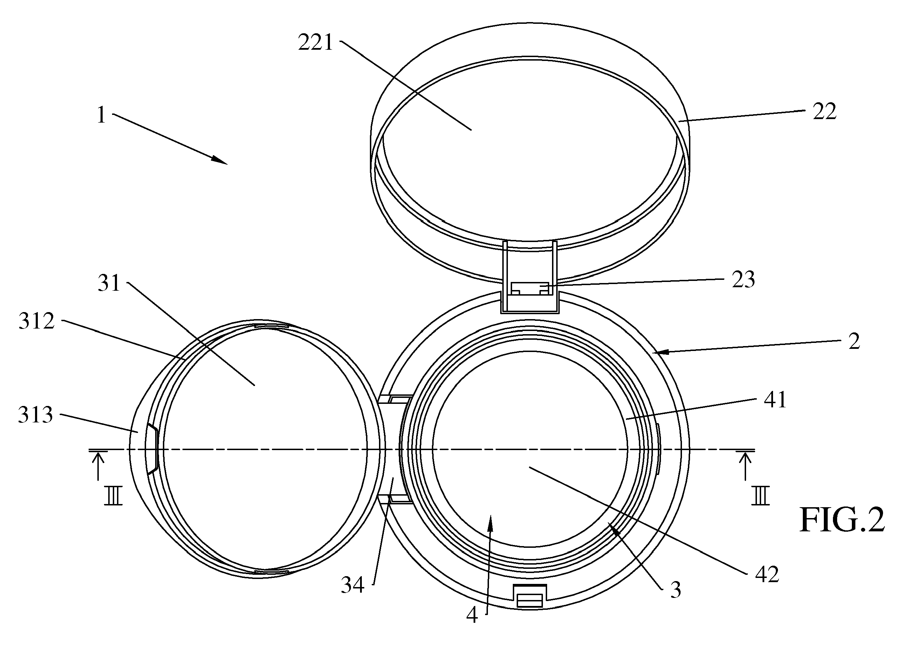

Figure 2 shows a top plan view of the container of cosmetic products;

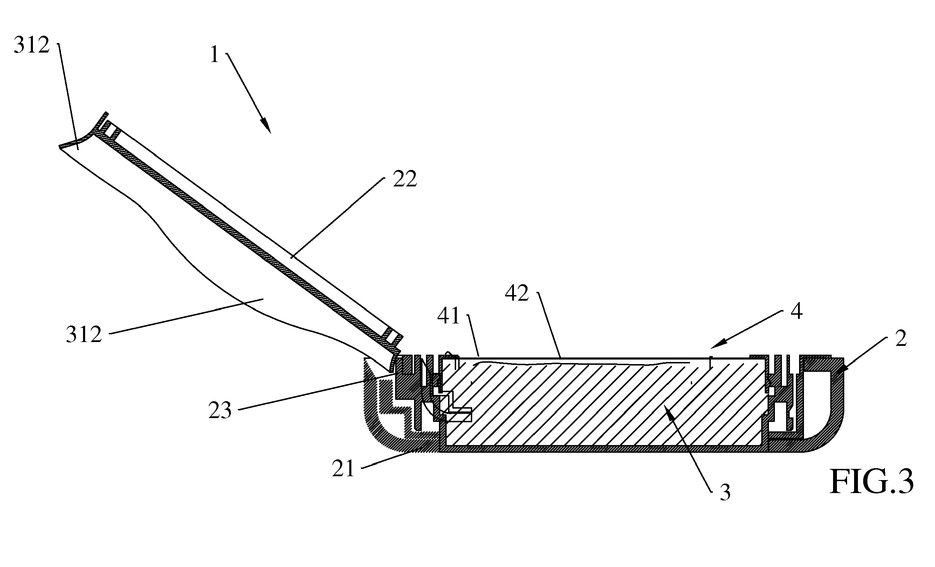

Figure 3 shows a sectional view of the container for cosmetic products according to the section line III-III in figure 2.

A container 1 comprises a support 2, a tank 3, an insert 4 and a sponge 5.

Support 2 is circular in shape, and comprises a base 21 and a lid 22 attached to said base 21 by means of a hinge 23.

Base 21 of support 2 is provided with a through opening 24, concentric with respect to base 21 and adapted to accommodate tank 3. Lid 22 of support 2 is a cap with a diameter equal to the diameter of base 21. A notch 25 provided in lid 22 is adapted to mate with a hook 26 provided on base 21 of said support 2. Hook 26 and notch 25 are adapted to keep base 21 attached to lid 22. Lid 22 comprises a mirror 221 fixed inside the cap.

Tank 3 has a circular base and has a cover 31 circular in shape and with the same diameter as tank 3 is attached to tank 3 by means of a hinge 34. Cover 31 comprises an edge 312, positioned in an outer portion of the cover, opposite the contact portion with tank 3. Edge 312 is provided with a projection 313, placed in opposite position with respect to the closing hinges 34, adapted to facilitate the lifting of cover 31.

Insert 4 comprises a support ring 41 and a flexible grid consisting of woven fabric, preferably a canvas 42. Said canvas 42 is attached to the support ring 41 by means of glue or by hot welding. Insert 4 is adapted to be inserted in tank 3.

Canvas 42 consists of polyamide, polyester or elastane, or a mixture thereof. Preferably, canvas 42 consists of a mixture containing 50% elastane and 50% polyamide. Preferably, 40% elastane and 60% polyamide.

Even more preferably, canvas 42 consists of the Thecno Light fabric of the company Nastri-Tex consisting of a mixture of 74% polyamide and 26% elastane, having an absorption capacity ranging from 1 to 100% according to the Test method UNI EN ISO 9073-6:2004.

Tank 3 is adapted to contain a cosmetic product consisting of a cosmetic formulation and at least one superabsorbent polymer.

The cosmetic formulation used in the present invention is an oil in water (O/W) emulsion or a water in oil (W/O) emulsion or a silicone in water (S/A) emulsion or a water in silicone (A/S) emulsion, or an anhydrous formulation or an aqueous solution or suspension.

The cosmetic formulation contains water in a percentage from 0 to 90% by weight with respect to the cosmetic formulation. Preferably from 0 to 85%.

Said cosmetic formulation may contain non-ionic, anionic, cationic or amphoteric emulsifiers in a percentage comprised between 0 and 20% by weight with respect to the cosmetic formulation. Preferably between 0.1 and 10%. The emulsifiers selected for the present invention have an HLB (hydrophilic-lipophilic balance) comprised between 1 and 20, preferably between 1 and 14.

The cosmetic formulation comprises oils selected from oils of all natures, hydrocarbons, esters, ethers, natural and synthetic triglycerides, silicone oils and polymers and resins. The oil content is comprised between 0.1 and 95% by weight with respect to the cosmetic formulation, preferably between 0.5 and 85%.

Sunscreen filters may be used for the preparation of the cosmetic formulation, such as organic or inorganic sunscreen filters with SPF (sun protection factor) of between 6 and 50+. The sunscreen filter percentage is comprised between 1 and 50% by weight with respect to the cosmetic formulation, preferably between 2 and 40%.

The cosmetic formulation further comprises pigments selected from titanium dioxide and iron oxides, lacquers and cosmetic dyes. The pigment percentage is comprised between 1% and 35% by weight with respect to the cosmetic formulation, preferably between 3 and 25%.

Texturizing powders selected from texturizing powders of silicone, acrylic, polymeric silica, mica and talc nature are added to the cosmetic formulation in a percentage comprised between 1 and 15% by weight with respect to the cosmetic formulation, preferably between 1 and 10%.

Superabsorbent polymers, sometimes referred to as superabsorbent polymer spheres, are crystals that absorb water or powder mud, have many practical applications. Superabsorbent polymers are used in the manufacture of diapers as solidification means of the products. Hospitals use them to consolidate and bio-hazardous waste and dispose of it in suction containers.

Superabsorbent polymers are a convenient alternative to other known absorbent materials. They absorb more liquid, by weight, than organic sorbents, are able to store the liquid under pressure and resist biodegradation.

Said superabsorbent polymers may have a water absorption capacity from 20 to 2000 times their own weight (i.e. 20 g to 2000 g of water absorbed per gram of absorbing polymer), preferably between 30 and 1500 times and even better from 50 to 1000 times. These water absorption characteristics are defined normal temperature conditions (25 °C) and pressure conditions (760 mmHg, i.e. 100,000 Pa) for distilled water.

The superabsorbent polymer used for the preparation of the cosmetic product of the present invention is in the form of particles. Preferably, the superabsorbent polymer has, in the dry or hydrated state, an average size greater than or equal to 100µm, preferably greater than or equal to 150µm, ranging for example from 150 to 1000µm, preferably from 150 to 850µm.

The average particle size corresponds to the weighted average diameter (D50) measured with laser particle size analysis or another equivalent method known by the those skilled in the art.

Superabsorbent polymers are now commonly made by the polymerization of acrylic acid mixed with sodium hydroxide in the presence of an initiator to form a sodium salt of polyacrylic acid (sometimes referred to as sodium polyacrylate). This polymer is the most common type of superabsorbent polymer currently made.

Other materials are also used to make a polymer superabsorbent, such as polyacrylamide copolymer, ethylene maleic anhydride copolymer, cross-linked carboxymethyl cellulose, polyvinyl alcohol copolymers, cross-linked polyethylene oxide, and starch polyacrylonitrile graft copolymer, among others. The latter is one of the oldest forms of superabsorbent polymers created.

Preferably, the superabsorbent polymers used in the present invention are in the form of spherical particles.

The superabsorbent polymers used in the present invention are selected from:
- a cross-linked polyacrylic acid sodium salt, such as those sold under the trade names FAVOR PAC 230 of the company Evonik, PREMIUM SAP of the company Zappatec, AQUA KEEP SA60S, AQUA KEEP SA60NSX II, AQUA KEEP SA60N TYPE II, AQUA KEEP CA300N, AQUA KEEP 10SH, AQUA KEEP 10SH PF, AQUA KEEP CA180N, AQUA KEEP 10SH-NF of the company Sumimoto, Sanwet 770H, of the company San-Dia Polymers, Hysorb, BASF 2300, BASF 7050, BASF 2260, BASF ASAP 2000 of the company BASF, Kolon GS3400 of the company Kolon Chemical, DryTech 2035M of the company Dry Tech, DOW S100R of the company Dow Chemical, CREALBLOC SIS, CABLOC, FIRESORB e STOCKOSORB of the company Evonik, HI SWELL of the company Song Wong, Activsoft MS100 of the company Innospec Performance Chemicals, Acusol AD-7 Polymer of the company The Dow Chemical Company, Aronvins of the company Nihon Junyaku Company, Cosmedia SP of the company BASF Personal Care and Nutrition, Covacryl Ac, Covacryl J22, Covacryl MV60, Covacryl RH of the company Sensient Cosmetic Technologies, Flocare CGEL 100, Flocare DP/PSD 100, Flocare G300, Flocare G800 of the company SNF SAS, Makimousse SV12, Makimousse SV 25 of the company Daito Kasei Kogyo, RapiThix A-100 of the company Ashland, Rheogic 250H, Rheogic 270, Rheogic 260H of the company Toagosei, Rheosol AP of the company Rheolabs, Rhesperse RM100 of the company Innospec Performance Chemicals, Sanfresh ST-500-MPSA of the company Risera Co., AQUA KEEP 10SH-NFC of the company Sumimoto Seika;
- starches grafted with an acrylic polymer (homopolymer or copolymer), in particular with sodium polyacrylate;
- hydrolyzed starches grafted with an acrylic polymer (homopolymer or copolymer), in particular acryloacrylamide/sodium acrylate copolymer, starch-based polymers, rubber and cellulose derivative, such as the product containing starch, guar gum and sodium carboxymethylcellulose, sold under the trade name Lysorb 220 by the company Lysac,
- mixtures of the polymers listed above.

The superabsorbent polymers used in the present invention can be cross linked or non-cross-linked. They are preferably selected from cross-linked polymers.

The superabsorbent polymers used in the present invention are preferably cross-linked acrylic homopolymers or copolymers which are preferably neutralized, and which are in the form of particles.

Preferably, the superabsorbent polymer is selected from cross-linked sodium polyacrylates, preferably in the form of particles with an average size (or mean diameter) greater than or equal to 100 microns, more preferably in the form of spherical particles.

These polymers are capable of absorbing an amount of water according to their weight ranging from 8 gr/gr to 400 gr/gr, i.e. from 8 to 400 times their weight. The percentage of polymer used ranges from a minimum of 0.01% to a maximum of 20% by weight with respect to the total weight of the cosmetic product and depends on the viscosity of the formula used: the higher the viscosity of the formula, the less polymer is used. Before being added to the cosmetic product, said superabsorbent polymer is hydrated with preserved water (containing any preservative system), the amount of water can range from a minimum of 30% to a maximum of 75% by weight with respect to the formula.

After the complete absorption of water, the cosmetic formulation of interest is added by simple stirring until complete homogenization of the emulsion.

Canvas 42 is adapted to absorb the cosmetic product, being in direct contact with said cosmetic product. Tank 3 of container 1 is adapted to contain an amount of cosmetic product adapted to impregnate canvas 42. Canvas 42 is adapted to transfer the liquid portion of the cosmetic product and not to filter the superabsorbent polymers so that they remain into tank 3.

After a contact with canvas 42, using sponge 5 or another means, the cosmetic product can be applied to the skin.

The following examples are intended to clarify the present invention without limiting it in any way.

### Example 1

### Diagram of the general formula of cosmetic product with a superabsorbent polymer

| | | Name (CTFA) | Quantity % |
|---|---|---|---|
| **PHASE A** | | | |
| | SUPERABSORBENT POLYMER | SODIUM POLYACRYLATE | 0.01 - 20 |

| **PHASE B** | | | |
|---|---|---|---|
| | | WATER | 30 - 75 |
| | | POTASSIUM SORBATE | 0.1 - 0.5 |
| | | SODIUM BENZOATE | 0.1 - 0.5 |

| **PHASE C** | | | |
|---|---|---|---|
| | COSMETIC FORMULATION | | 15 - 60 |

### Example 2

### Example of diagram of general formula

| Type of raw material | Description | Percentage range |
|---|---|---|
| WATER | - | 0 - 85% |
| EMULSIFIER | Non-ionic, anionic, cationic or amphoteric emulsifiers with HLB from 1-14 | 0.1 - 10 % |
| OILS | Cosmetic oils of all natures, hydrocarbons, esters, ethers, natural and synthetic triglycerides, silicone oils and polymers and resins | 0.5 - 85% |
| SUNSCREENS | Organic or inorganic sunscreens, for sun protection *claim* from SPF 6 TO SPF 50+ | 2 - 40 % |
| PIGMENTS | Titanium dioxide and iron oxide for all color shades | 3 - 25 % |
| TEXTURIZING POWDERS | Silicone, acrylic, polymeric silica, mica, talc-based texturizing powders. | 1 - 10 % |

### Example 3

### Example of cosmetic product with a superabsorbent polymer

| | Trade name | Name (CTFA) | Quantity % |
|---|---|---|---|
| **PHASE A** | | | |
| | SUPERABSORBENT POLYMER | SODIUM POLYACRYLATE | 6.250 |

| **PHASE B** | | | |
|---|---|---|---|
| | | WATER | 62.000 |
| | | POTASSIUM SORBATE | 0.250 |
| | | SODIUM BENZOATE | 0.250 |

| **PHASE C** | | | |
|---|---|---|---|
| | COSMETIC FORMULATION | | 31.250 |

### Example 4

### Example of cosmetic formulation used

| | Trade name | Name (CTFA) | Quantity % |
|---|---|---|---|
| **PHASE A** | | | |
| | | WATER | 71.970 |
| | | TETRASODIUM EDTA | 0.100 |
| | | CHLORPHENESIN | 0.270 |
| | | CITRIC ACID | 0.060 |
| | | GLYCERIN | 2.000 |
| | | BUTYLENE GLYCOL | 2.000 |
| | | POLYSORBATE 20 | 2.500 |

| **PHASE B** | | | |
|---|---|---|---|
| | | TITANIUM DIOXIDE (CI 77891) AND PEG-12 DIMETHICONE | 12.300 |
| | | IRON OXIDES (CI 77492) AND PEG-12 DIMETHICONE | 1.800 |
| | | IRON OXIDES (CI 77491)AND PEG-12 DIMETHICONE | 0.330 |
| | | IRON OXIDES (CI 77499) AND PEG-12 DIMETHICONE | 0.100 |

| **PHASE C** | | | |
|---|---|---|---|
| | | NYLON-12 | 5.000 |

| **PHASE D** | | | |
|---|---|---|---|
| | | Microcrystalline Cellulose (and) Cellulose Gum | 0.500 |

| **PHASE E** | | | |
|---|---|---|---|
| | | PHENOXYETHANOL | 0.700 |
| | | ETHYLHEXYLGLYCERIN | 0.360 |

### Example 5

### Example of oil-in-water emulsion used as a cosmetic formulation.

| | Trade name | Name (CTFA) | Quantity % |
|---|---|---|---|
| PHASE A 25 °C | | | |
| | | WATER | 56.450 |
| | | GLYCERIN | 3.000 |
| | | BUTYLENE GLYCOL | 2.000 |
| | | PEG-12 DIMETHICONE | 1.500 |
| | | POLYSORBATE 60 | 0.500 |

| PHASE B | | | |
|---|---|---|---|
| | | SODIUM POLYACRYLATE | 0.250 |
| | | XANTHAN GUM | 0.150 |

| PHASE C | | | |
|---|---|---|---|
| | | TITANIUM DIOXIDE (CI 77891) AND PEG-12 DIMETHICONE | 12.000 |
| | | IRON OXIDES (CI 77492) AND PEG-12 DIMETHICONE | 2.400 |
| | | IRON OXIDES (CI 77491)AND PEG-12 DIMETHICONE | 0.450 |
| | | IRON OXIDES (CI 77499) AND PEG-12 DIMETHICONE | 0.150 |

| PHASE D | | | |
|---|---|---|---|
| | | CAPRYLIC/CAPRIC TRIGLYCERIDE | 8.000 |
| | | SQUALANE | 5.000 |
| | | ISONONYL ISONONANOATE | 6.500 |
| | | DIMETHICONE | 0.500 |

| PHASE E | | | |
|---|---|---|---|
| | | ETHYLHEXYLGLYCERIN | 0.350 |
| | | PHENOXYETHANOL | 0.800 |

### Example 6

### Example of water-in-silicone emulsion used as a cosmetic formulation.

| | Trade name | Name (CTFA) | Quantity % |
|---|---|---|---|
| **PHASE A** | | | |
| | | | 4.000 |
| | | STEAROYL INULIN | 1.000 |

| **PHASE B** | | | |
|---|---|---|---|
| | | DIMETHICONE AND TRISILOXANE | 9.100 |
| | | CYCLOPENTASILOXANE and CAPRYLYC DIMETHICONE ETHOXY GLUCOSIDE | 10.000 |
| | | CETYL PEG/PPG-10/1 DIMETHICONE AND POLYGLYCERYL-4 ISOSTEARATE AND HEXYL LAURATE | 0.500 |

| **PHASE C** | | | |
|---|---|---|---|
| | | ETHYLHEXYL METHOXYCINNAMATE | 2.000 |

| **PHASE D** | | | |
|---|---|---|---|
| | | TITANIUM DIOXIDE (nano) | 1.500 |

| **PHASE E** | | | |
|---|---|---|---|
| | | TITANIUM DIOXIDE AND DIMETHICO NE | 12.600 |

| **PHASE F** | | | |
|---|---|---|---|
| | | DIMETHICONE AND TRISILOXANE | 4.980 |
| | | | 4.000 |
| | | DIMETHICONE AND CAPRYLYL METHICONE AND POLYSILICONE-11 | 4.000 |
| | | CETYL PEG/PPG-10/1 DIMETHICONE AND POLYGLYCERYL-4 ISOSTEARATE AND HEXYL LAURATE | 0.500 |
| | | | 1.750 |
| | | | 0.290 |
| | | | 0.120 |
| | | | 0.240 |

| | Trade name | Name (CTFA) | Quantity % |
|---|---|---|---|
| **PHASE G** | | | |
| | | HDI/TRIMETHYLOL HEXYLLACTONE CROSSPOLYMER AND SILICA | 4.000 |
| | | ETHYLENE/ACRYLIC ACID COPOLYMER | 4.000 |

| **PHASE H** | | | |
|---|---|---|---|
| | | CAPRYLYL GLYCOL, PHENOXYETHANOL, HEXYLENE GLYCOL | 1.000 |
| | | | 0.010 |
| | | | |
| | | ETHYLHEXYL PALMITATE AND SILICA DIMETHYL SILYLATE AND BUTYLENE GLYCOL AND CAPRYLYL GLYCOL AND PHENOXYETHANOL AND HEXYLENE GLYCOL AND SODIUM HYALURONATE | 0.010 |
| **PHASE I** | | | |
| | | WATER | 27.900 |
| | | PEG 4 | 4.000 |
| | | SODIUM CHLORIDE | 1.000 |
| | | POTASSIUM SORBATE | 0.400 |

| **PHASE L** | | | |
|---|---|---|---|
| | | ALCOHOL | 1.000 |

| **PHASE M** | | | |
|---|---|---|---|
| | | WATER AND UREA AND YEAST AMINO ACIDS AND TREHALOSE AND INOSITOL AND TAURINE AND BETAINE | 0.100 |

### Example 7

### Example of silicone-in-water emulsion used as a cosmetic formulation.

| | Trade name | Name (CTFA) | Quantity % |
|---|---|---|---|
| **PHASE A** | | | |
| | | WATER | 52.930 |
| | | BUTYLENE GLYCOL | 3.000 |
| | | GLYCERIN | 2.000 |
| | | DISODIUM EDTA | 0.100 |
| | | SODIUM BENZOATE | 0.400 |

| **PHASE B** | | | |
|---|---|---|---|
| | | XANTHAN GUM | 0.100 |
| | | SODIUM POLYACRYLATE | 0.300 |

| **PHASE C** | | | |
|---|---|---|---|
| | | TITANIUM DIOXIDE (CI 77891) AND PEG-12 DIMETHICONE | 11.380 |
| | | IRON OXIDES (CI 77492) AND PEG-12 DIMETHICONE | 2.100 |
| | | IRON OXIDES (CI 77491)AND PEG-12 DIMETHICONE | 0.400 |
| | | IRON OXIDES (CI 77499) AND PEG-12 DIMETHICONE | 0.140 |

| **PHASE D** | | | |
|---|---|---|---|
| | | PEG-9 DIMETHICONE | 5.000 |
| | | DIMETHICONE AND DIMETHICONE/PEG-10/15 CROSSPOLYMER | 1.500 |
| | | DIMETHICONE AND DIMETHICONE CROSSPOLYMER | 2.000 |
| | | DIMETHICONE AND DIMETHICONE/VINYL DIMETHICONE CROSSPOLYMER | 2.500 |
| | | DIMETHICONE | 6.000 |
| | | DIMETHICONE | 4.000 |
| | | ETHYLHEXYLGLYCERIN | 0.350 |
| | | PHENOXYETHANOL | 0.800 |

| **PHASE E** | | | |
|---|---|---|---|
| | | DIPHENYL DIMETHICONE/ VINYL DIPHENYL DIMETHICONE/ SILSESQUIOXANE CROSSPOLYMER | 1.000 |
| | | VINYL DIMETHICONE/METHICONE SILSESQUIOXANE CROSSPOLYMER | 1.000 |
| | | | 2.500 |
| | | HYDRATED SILICA | 0.500 |

### Example 8

### Example of water-in-oil emulsion used as a cosmetic formulation.

| | Trade name | Name (CTFA) | Quantity % |
|---|---|---|---|
| ***PHASE B 60 °C*** | | | |
| | | BUTYLENE GLYCOL DICAPRYLATE/DICAPRATE | 4.700 |
| | | DIMETHICONE | 5.640 |
| | | ISODODECANE | 15.000 |
| | | ETHYLHEXYL METHOXYCINNAMATE | 5.000 |
| | | PEG-30 DIPOLYHYDROXYSTEARATE | 3.500 |
| | | SORBITAN SESQUIOLEATE | 1.000 |

| ***PHASE C LAMINAR*** | | | |
|---|---|---|---|
| | | ISOHEXADECANE | 4.200 |
| | | SORBITAN SESQUIOLEATE | 0.500 |
| | | TITANIUM DIOXIDE | 3.000 |
| | | TITANIUM DIOXIDE (CI 77891) AND DISODIUM STEAROYL GLUTAMATE AND ALUMINUM HYDROXIDE | 7.940 |
| | | IRON OXIDES (CI 77492) AND DISODIUM STEAROYL GLUTAMATE AND ALUMINUM HYDROXIDE | 1.920 |
| | | IRON OXIDES (CI 77491) AND DISODIUM STEAROYL GLUTAMATE AND ALUMINUM HYDROXIDE | 0.360 |
| | | IRON OXIDES (CI 77499) AND DISODIUM STEAROYL GLUTAMATE AND ALUMINUM HYDROXIDE | 0.140 |

| ***PHASE D*** | | | |
|---|---|---|---|
| | | SILICA | 4.000 |
| | | POLYMETHYLSILSESQUIOXAN E | 3.000 |
| | | METHYL METHACRYLATE CROSSPOLYMER | 3.000 |

| ***PHASE E*** | | | |
|---|---|---|---|
| | | WATER | 25.000 |
| | | BUTYLENE GLYCOL | 3.000 |
| | | POTASSIUM SORBATE | 0.300 |
| | | SODIUM DEHYDROACETATE | 0.300 |
| | | SODIUM CHLORIDE | 1.000 |

| ***PHASE F*** | | | |
|---|---|---|---|
| | | ALCOHOL | 7.500 |

Advantageously, the use of the superabsorbent polymers and of the canvas allows also to manage fluid cosmetic preparations contained in portable containers.

Another advantage is the ease of application of the cosmetic product that allows an application without the risk of getting dirty.

A further advantage is that the canvas allows the passage of only the fluid part of the cosmetic product, retaining the superabsorbent polymers in the tank.

Yet another advantage is that the superabsorbent polymers allow the preparation of long-lasting cosmetic formulations.

An additional advantage is that the canvas allows an even distribution of the product, without waste and ensuring a correct application of the product.

## Claims

1. Container (1) of cosmetic products, comprising a support (2), a tank (3) and an insert (4) **characterized in that** said insert (4) comprises a flexible grid formed of woven fabric, preferably a canvas (42), the reservoir (3) contains a cosmetic product having a cosmetic formulation mixed with a superabsorbent polymer, said canvas (42) put in contact with said cosmetic product and suitable for impregnating with said cosmetic product by means of pressure application with a sponge (5).

2. Container (1) according to claim 1, **characterized in that** the canvas (42) is constituted by polyamide or polyester or elastane or a mixture of these.

3. Container (1) according to claims 1 or 2, **characterized in that** the canvas (42) is constituted by a mixture containing 60% elastane and 40% polyamide.

4. Container (1) according to claims 1-3, **characterized in that** the canvas (42) is constituted by a mixture containing 26% elastane and 74% polyamide.

5. Container (1) according to claims 1, **characterized in that** the superabsorbent polymer is selected from salts of sodium polyacrylic or a starch grafted with an acrylic polymer (homopolymer or copolymer), or between hydrolyzed starches grafted with a polymer acrylic, in particular acrylate copolymer acrylo-acrylamide/sodium, or between polymers of starch-based rubber and on cellulose derivative, products containing starch, guar gum and sodium carboxymethylcellulose.

6. Container (1) according to claim 5, **characterized in that** the superabsorbent polymer is selected from cross-linked sodium polyacrylates, preferably in the form of particles with an average size (or mean diameter) greater than or equal to 100 microns in the dry state or not hydrated.

7. Container (1) according to claims 5 or 6, **characterized in that** the superabsorbent polymer is selected from cross-linked sodium polyacrylates, preferably in the form of particles with an average size (or mean diameter) of between 150 and 1000µm in the dry state or not hydrated.

8. Container (1) according to claims 5-7, **characterized in that** the superabsorbent polymer is selected from cross-linked sodium polyacrylates, preferably in the form of particles with an average size (or mean diameter) of between 150 and 850µm in the dry state or not hydrated.

9. Container (1) according to claims 5-8, **characterized in that** the superabsorbent polymer is selected from cross-linked sodium polyacrylates, preferably in the form of spherical particles.

10. Container (1) according to any one of the preceding claims, **characterized in that** the cosmetic formulation is an emulsion oil in water (O/W) or water in oil emulsion (W/O) emulsion of silicone in water (S/A) or an emulsion of water in silicone (A/S), or an anhydrous formulation or aqueous solutions or suspensions.

11. Container (1) according to any one of the preceding claims, **characterized in that** the cosmetic formulation contains water in a percentage between 0 and 85%.

12. Container (1) according to any one of the preceding claims, **characterized in that** the percentage of superabsorbent polymer used varies from a minimum of 0.01% to a maximum of 20% by weight with respect to the total weight of the cosmetic product.
